# FASCICULE DE BREVET EUROPEEN

(11) **EP 0 467 767 B1**
(45) Date de publication et mention de la délivrance du brevet: **21.09.1994**
(21) Numéro de dépôt: 91401966.6
(22) Date de dépôt: 15.07.1991
(51) Int. Cl.: A61K 7/13, A61K 7/00, A61K 7/021, C09C 3/08, C09C 1/36

(54) **Produit à base de particules lamellaires comportant un pigment mélanique et son procédé de préparation**
Produkte auf Basis von Melaninpigment enthaltenden plättchenförmigen Partikeln und Verfahren zu ihrer Herstellung
Product based on flaky particles containing melanine pigment and process for their preparation

(30) Priorité: 16.07.1990 FR 9009053
(43) Date de publication de la demande: 22.01.1992
(73) Titulaire: L'OREAL, 75008 Paris (FR)
(72) Inventeur: Andrean, Hervé, F-75014 Paris (FR); Junino, Alex, F-93180 Livry-Gargan (FR)
(74) Mandataire: Casalonga, Axel

(56) Documents cités:
- EP-A- 0 191 292
- EP-A- 0 220 617
- GB-A- 2 207 153

## Description

La présente invention est relative à un produit sous forme de particules lamellaires, minérales ou organiques, comportant des pigments mélaniques, à son procédé de préparation et à son utilisation, notamment dans le domaine de la cosmétique, pour la coloration des cheveux, le maquillage des poils et/ou de la peau, la protection de l'épiderme humain contre le rayonnement UV.

La couleur des cheveux, de la peau et des poils d'origine humaine provient principalement des pigments mélaniques sécrétés par les mélanocytes.

Ces pigments, d'origine naturelle, comprennent en particulier des pigments noirs ou bruns qu'on appelle les eumélanines.

Leur biosynthèse naturelle s'effectue en plusieurs étapes par polymérisation des produits d'oxydation d'un acide aminé : la tyrosine et l'un de ces produits d'oxydation est le 5,6-dihydroxyindole qui polymérise à son tour en eumélanine.

La demanderesse a décrit, dans les demandes de brevets et brevets antérieurs, différents procédés permettant de teindre les cheveux humains ou la peau avec du 5,6-dihydroxyindole ou ses dérivés, en mettant en oeuvre différents systèmes d'oxydation. Les colorants ainsi formés se fixent ou pénètrent dans le substrat kératinique.

On souhaite, à certaines occasions, pouvoir conférer aux cheveux une coloration qui puisse éventuellement être enlevée rapidement.

On utilise, par ailleurs, dans les compositions de maquillage pour la peau, les poils, les cils ou les sourcils, des pigments à base de composés métalliques tels que par exemple les oxydes de fer, noir et brun. Ces pigments ne sont cependant pas totalement inoffensifs et on rechercbe de ce fait des pigments susceptibles de présenter moins de problèmes dans leur application cosmétique.

La demanderesse a déjà proposé dans la demande FR 2 618 069, l'utilisation pour la coloration de la peau, des poils, des cils et sourcils ou des cheveux, d'une poudre composée de particules minérales ultrafines de diamètre moyen inférieur à 20 um, portant sur leur surface un pigment mélanique résultant de la polymérisation oxydative de composés indoliques 5,6-disubstitués.

La demanderesse vient de découvrir qu'il était possible de préparer in vitro un produit sous forme à'une poudre formée de particules lamellaires, minérales ou organiques, comportant un ou plusieurs pigments mélaniques.

Elle a découvert que l'utilisation de particules lamellaires était particulièrement avantageuse dans la mesure où ces particules, une fois introduites dans un milieu cosmétiquement acceptable, se répartissaient bien dans la composition qui s'étale facilement sur les cheveux ou la peau et présente un pouvoir couvrant important.

Par ailleurs, on a constaté qu'elles présentaient un coefficient d'absorption des radiations ultraviolettes particulièrement intéressant par rapport aux produits connus jusqu'à présent.

On appelle "particules lamellaires" conformément à l'invention, des particules se présentant sous forme de feuillets éventuellement stratifiés.

Ces feuillets se caractérisent par une épaisseur plus faible que la plus grande dimension. De préférence, le rapport entre la plus grande dimension et l'épaisseur est compris entre 2 et 100.

On appelle "pigment mélanique" le pigment formé par oxydation du 5,6-dihydroxyindole éventuellement associé à l'acide 2-carboxylique 5,6-dihydroxyindole.

Par analogie et simplification on appellera "pigment mélanique", le pigment formé par oxydation des différents composés de formule (I) définie ci-après.

La présente invention a donc pour objet une poudre constituée de particules de structure lamellaire, minérale ou organique, comportant des pigments mélaniques.

Un autre objet de l'invention est constitué par la préparation d'une telle poudre.

L'invention a également pour objet l'application cosmétique de telles poudres, notamment dans les produits de maquillage de la peau, des poils (cils et sourcils), la protection de l'épiderme humain contre le rayonnement UV et la coloration des cheveux.

D'autres objets de l'invention apparaîtront à la lecture de la description et des exemples qui suivent.

Le produit conforme à l'invention est essentiellement caractérisé par le fait qu'il se présente sous forme d'une poudre constituée de particules de structure lamellaire, minérales ou organiques, dont la dimension la plus grande est inférieure à 50 microns et comportant dans ou sur la structure lamellaire, un pigment mélanique synthétique formé in situ.

Le pigment mélanique résulte de l'oxydation d'au moins un colorant indolique, répondant à la formule: 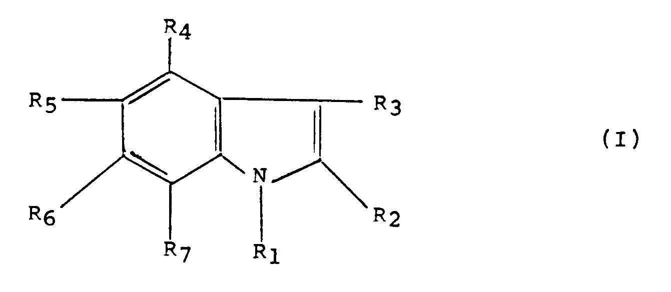
dans laquelle :
R₁ et R₃ désignent, indépendamment l'un de l'autre, un atome d'hydrogène ou un groupe alkyle en C₁-C₄;
R₂ représente un atome d'hydrogène, un groupement alkyle en C₁-C₄, un groupement carboxyle ou un groupement alcoxy(C₁-C₄)carbonyle;
R₄ et R₇ désignent, indépendamment l'un de l'autre, un atome d'hydrogène, un groupement hydroxy, un groupe alkyle en C₁-C₄, amino, alcoxy(C₁-C₄), acyl (C₂-C₄)oxy, acyl(C₂-C₄)amino;
R₅ désigne hydrogène, un groupement hydroxy, alcoxy(C₁-C₄), alkyle(C₁-C₄), halogène, amino, acyl (C₂-C₁₄)oxy, acyl(C₂-C₄)amino, triméthylsilyloxy;
R₆ désigne hydrogène, un groupement hydroxy, alcoxy(C₁-C₄), amino, acyl(C₂-C₄)oxy, acyl(C₂-C₄) amino, triméthylsilyloxy, hydroxyalkyl(C₂-C₄)amino;
R₅ et R₆ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy; au moins l'un des groupements R₄ à R₇ représente un groupement OZ ou NHR, un seul au plus des groupements R₄ à R₇ désignant NHR;
et au plus deux des groupements R₄ à R₇ désignent OZ, dans le cas où Z désigne hydrogène, ces groupements sont en position 5 et 6;
et au moins un des groupements R₄ à R₇ représente hydrogène, dans le cas où un seul de ces groupements désigne hydrogène, un seul groupement parmi R₄ à R₇ désigne alors NHR ou OZ, et les autres groupements désignent alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants.

Les colorants indoliques de formule (I) sont choisis de préférence parmi le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxy indole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxy carbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, 5-aminoindole, 6-aminoindole, 7-amino indole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole.

Le 5,6-dihydroxyindole et le 6-hydroxyindole sont particulièrement préférés.

Les particules de structure lamellaire sont choisies en particulier parmi les produits suivants : la L-Lauroyllysine telle que le produit vendu sous la dénomination AMIHOPE L.L par la Société AJINOMOTO; les microparticules de céramique éventuellement recouvertes de poudre de zirconium telles que les produits vendus sous les appellations TORAYCERAM ZP 550 et ZP 4000 par la Société TORAY; le dioxyde de titane lamellaire tel que les produits vendus sous les dénominations LUXELEN SILK D et LUXELEN SS par la Société SUMITOMO, le talc lamellaire, le nitrure de bore tel que les produits vendus sous les dénominations Nitrure de bore SF ou SHP par les Sociétés WACKER et KAWASAKI; le mica lamellaire tel que le produit vendu sous la dénomination MICA CONCORD 1000 par la Société SCIAMA; l'oxychlorure de bismuth tel que le produit vendu sous la dénomination PEARL GLO par la Société MALLINCKRODT; l'oxyde de fer transparent rouge tel que le produit vendu sous la dénomination CAPPOXYT 4435 B par la Société CAPPELLE.

La dimension des particules de structure lamellaire utilisées conformément à l'invention, est de préférence inférieure à 50 microns et en particulier inférieure à 25 microns. Leur dimension est généralement supérieure à 0,5 micron. Elle est comprise en particulier entre 1 et 20 microns. Ces particules ont une épaisseur généralement supérieure à 0,01 micron. Comme indiqué plus haut, ces particules lamellaires peuvent se présenter sous forme d'une structure stratifiée.

Le produit conforme à l'invention est préparé de préférence selon un procédé consistant à mélanger à l'air et à température de préférence ambiante, pouvant aller jusqu'à 100°C, le composé indolique de formule (I) et les particules de structure lamellaire définies ci-dessus, dans un milieu essentiellement non solvant des particules lamellaires.

L'oxydation du composé indolique de formule (I) peut s'effectuer en milieu aqueux ou eau-solvant, à l'air, à l'air en présence d'un agent alcalin et/ou d'un catalyseur métallique d'oxydation.

Un catalyseur métallique d'oxydation préféré est constitué par l'ion cuivrique.

L'oxydation peut également s'effectuer en mettant en oeuvre le peroxyde d'hydrogène en présence d'un agent alcalin, tel que de préférence l'ammoniaque, ou en présence d'un ion iodure, l'iodure étant de préférence l'iodure d'un métal alcalin, alcalino-terreux ou d'ammonium.

On peut également procéder à l'oxydation en utilisant l'acide periodique et ses sels hydrosolubles et dérivés, les permanganates et bichromates, tels que de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont notamment le cérium et des oxydants organiques choisis parmi les ortho et parabenzoquinones, les ortho et parabenzoquinones monoimines ou diimines, les 1,2 et 1,4-naphtoquinones, les 1,2 et 1,4-naphtoquinones mono-ou diimines. Le sel d'acide periodique préféré est le periodate de sodium.

Il est possible d'activer les agents oxydants par un agent modificateur de pH.

Par exemple, lors de l'utilisation du système iodure/peroxyde hydrogène, on met en oeuvre de préférence un milieu alcalin, ce qui permet d'activer la réaction.

Le procédé particulièrement préféré consiste à utiliser un iodure alcalin, alcalino-terreux ou d'ammonium, du peroxyde d'hydrogène à un pH acide ou alcalin et dans ce cas il s'agit d'un milieu ammoniacal.

Le milieu réactionnel utilisé pour former le colorant sur et dans les particules à structure lamellaire, est un milieu essentiellement non solvant des particules à structures lamellaires considérées. Il est de préférence constitué par de l'eau et peut éventuellement être constitué par 'un mélange d'eau et de solvant(s). Le solvant est choisi de telle façon qu'il solubilise rapidement le composé indolique de formule (I).

Parmi ces solvants, on peut citer à titre d'exemple, les alcools inférieurs en C₁-C₄ tels que l'alcool éthylique, l'alcool propylique ou isopropylique, l'alcool tertiobutylique, les alkylène glycols tels que l'éthylèneglycol, le propylèneglycol, les alkyléthers d'alkylèneglycols tels que les éthers monométhylique, monoéthylique et monobutylique de l'éthylèneglycol, les monométhyléthers du propylène glycol et du dipropylèneglycol, et le lactate de méthyle.

Lorsque le milieu est constitué par un mélange eau/solvant(s), le(s) solvant(s) est (sont) présent(s) dans des concentrations comprises entre 0,5 et 90% en poids par rapport au poids total de la composition, en particulier entre 2 et 50% en poids et de préférence entre 2 et 20% en poids.

Leur nature est choisie et leur proportion est ajustée en fonction des critères de solubilité des dérivés indoliques de formule (I) et du critère d'insolubilité des particules lamellaires.

Dans le procédé de préparation des produits sous forme de particules conformes à l'invention, on utilise de préférence le composé indolique dans des proportions pondérales comprises entre 0,1 et 10% et de préférence entre 0,5 et 5% en poids par rapport au poids total du milieu réactionnel, la charge lamellaire représentant 0,05 à 35% en poids du milieu réactionnel, le reste du mélange réactionnel étant généralement constitué par de l'eau ou un mélange eau/solvant(s).

Les oxydants sont mis en oeuvre dans des quantités suffisantes pour former, par oxydation, le pigment mélanique.

Lorsqu'on utilise l'ion iodure pour former le pigment mélanique, celui-ci est utilisé de préférence dans des proportions comprises entre 0,07 et 4% et en particulier entre 0,7 et 3%, en observant un rapport colorant indolique/I⁻ compris entre 0,6 et 6 et plus particulièrement compris entre 3 et 4.

Les proportions sont déterminées par rapport au poids du milieu réactionnel.

La poudre sous forme de particules à structure lamellaire organique ou minérale et comportant le pigment mélanique tel que défini ci-dessus, peut être additionnée dans des supports cosmétiques classiques à une concentration comprise entre 0,1 et 35% en poids et de préférence entre 0,5 et 20% en poids par rapport au poids total de la composition pour conduire à des compositions cosmétiques protectrices de l'épiderme humain, des produits de maquillage tels que des cils, des sourcils ou de la peau, comme des fards à paupières, fards à joues, ligneurs encore appelés "eye-liners", des mascaras pour les cils et les sourcils ou encore des compositions tinctoriales pour cheveux. Ces supports cosmétiques sont connus en eux-mêmes.

Les compositions peuvent se présenter en particulier sous forme de lotion, de lotion épaissie, de gel, de crème, de lait, de poudre, de stick et éventuellement être conditionnées en aérosol et se présenter sous forme de mousse ou de spray.

Lorsque les compositions sont utilisées pour le maquillage de la peau, des cils et des sourcils, elles peuvent notamment se présenter sous forme solide ou pâteuse, anhydre ou aqueuse, comme des émulsions huile-dans-eau ou eau-dans-huile ou encore des suspensions. Ces compositions présentent l'avantage d'être stables et de présenter une bonne innocuité.

Lorsque les compositions sont utilisées pour la protection de l'épiderme humain contre le rayonnement UV, elles constituent des compositions dites "solaires " et elles peuvent se présenter sous forme de suspensions ou de dispersions dans des solvants ou des corps gras, ou encore sous forme d'émulsions telles que crèmes et laits, de pommades, de gels, de bâtonnets solides ou de mousses aérosols.

Dans tous les cas, lorsqu'elles sont utilisées sous forme d'émulsions, elles peuvent contenir en outre des agents tensio-actifs bien connus dans l'état de la technique, tels que des agents tensio-actifs anioniques, non-ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non ioniques, amphotères, ou leurs mélanges, des propulseurs, des agents alcalinisants ou acidifiants.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin Hydrogénée, l'huile de vaseline, l'huile de paraffine, l'huile de Purcellin.

Les cires sont choisies parmi les cires animales, fossiles, végétales, minérales ou de synthèse. On peut citer notamment les cires d'abeilles, les cires de Carnauba, de Candellila, de canne à sucre, du Japon, les ozokérites, la cire de Montan, les cires microcristallines, les paraffines.

Les compositions conformes à l'invention peuvent également contenir en plus des particules lamellaires comportant des pigments mélaniques, telles que définies ci-dessus, d'autres pigments généralement utilisés en cosmétique, notamment des pigments nacrés et/ou nacrants permettant de varier les colorations susceptibles d'être obtenues, ou d'augmenter la protection vis-à-vis du rayonnement ultraviolets. Dans le dernier cas, on utilise des pigments d'oxydes métalliques tels que les oxydes de titane, de zinc, de cérium ou de zirconium.

On utilise préférentiellement des "nanopigments", de diamètre moyen inférieur à 100 nm et de préférence compris entre 5 et 50 nm. Les nano-pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans COSMETICS and TOILETRIES, février 1990, Vol.105, pages 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Le milieu utilisé dans ces différentes compositions cosmétiques est un milieu essentiellement non solvant des particules à structure lamellaire minérales ou organiques, comportant le pigment mélanique.

On appelle milieu essentiellement non solvant, un milieu qui dissout moins de 1% en poids des particules lamellaires.

L'invention a également pour objet un procédé de teinture des cheveux, de maquillage de la peau, de la protection de l'épiderme humain contre les effets néfastes des rayonnements UV, mettant en oeuvre une poudre à base de particules à structure lamellaire organique ou minérale et comportant des pigments mélaniques telle que définie ci-dessus, cette poudre étant appliquée directement ou au moyen de compositions cosmétiques telles que définie ci-dessus.

Les exemples suivants sont destinés à illustrer l'invention.

### EXEMPLE 1

On solubilise 5 g (3,3 10⁻² moles) de 5,6-dihydroxyindole dans 100 ml de solution aqueuse à 0,1% d'ammoniac. On ajoute à ce mélange 45 g d'oxychlorure de bismuth vendu sous la dénomination PEARL GLO UVR 1086 par la Société MALLINCKRODT,, on agite 15 minutes la suspension puis on la porte à 80°C. On additionne en 15 minutes 28,77 g d'eau oxygénée contenant 2,3 g (6,7 10⁻² moles ) de peroxyde d'hydrogène en maintenant la température entre 80 et 85°C. L'addition terminée, on maintient la température à 80°C pendant 2 heures, puis on refroidit le milieu réactionnel à 10°C. On essore le produit et on le lave à l'eau. Après séchage, on obtient 48 g de poudre brun foncé.

### EXEMPLE 2

On solubilise 5 g (3,3 10⁻² moles) de 5,6-dihydroxyindole dans 100 ml de solution aqueuse à 0,1% d'ammoniac. On ajoute à ce mélange 45 g de nitrure de bore vendu sous la dénomination SH P2 par la Société KAWASAKI et on agite 15 minutes la suspension puis on la porte à 80°C. On additionne en 15 minutes 28,77 g d'eau oxygénée contenant 2, 3 g (6,7 10⁻² moles ) de peroxyde d'hydrogène en maintenant la température entre 80 et 85°C. L'addition terminée, on maintient la température à 80°C pendant 2 heures, puis on refroidit le milieu réactionnel à 10°C. On essore le produit et on le lave à l'eau. Après séchage, on obtient 48 g de poudre brun foncé.

### EXEMPLE 3

On solubilise 7 g (4,6 10⁻² moles) de 5,6-dihydroxyindole dans 140 ml de solution aqueuse à 0,1% d'ammoniac. On ajoute à ce mélange 63 g de mica vendu sous la dénomination MICA CONCORD 1000 par la Société SCIAMA, et on agite 15 minutes la suspension puis on la porte à 80°C. On additionne en 1 heure 24 g d'eau oxygénée contenant 3,12 g (9,2 10⁻² moles) de peroxyde d'hydrogène en maintenant la température entre 80 et 85°C. L'addition terminée, on maintient la température à 80°C pendant 1 heure, puis on refroidit le milieu réactionnel à 20°C. On essore le précipité et on le lave à l'eau. Après séchage, on obtient 70 g de poudre brun foncé.

### EXEMPLE 4

On solubilise 11,1 g de 5,6-dihydroxyindole dans 243 ml de solution aqueuse à 2% d'ammoniac. On ajoute à ce mélange 100 g d'oxyde de fer transparent rouge vendu sous la dénomination CAPPOXYT ROUGE 4435 B par la Société CAPPELLE et on chauffe la suspension à 80°C. On additionne en 30 minutes 28,57 g d'eau oxygénée à 110 volumes, diluée dans 84 g d'eau permutée, en maintenant la température entre 80 et 85°C. L'addition terminée, on maintient le chauffage pendant 2 h 30 minutes à 80-85°C, puis on refroidit le milieu réactionnel à 20°C. On essore le précipité et on le lave à l'eau. Après séchage, on obtient 108,2 g de poudre brun noir.

### EXEMPLES D'APPLICATION

### EXEMPLE 1

On prépare une émulsion solaire huile-dans-eau ayant la composition suivante :

| | |
|---|---|
| - Mélange d'alcool cétylstéarylique et d'alcool cétylstéarylique oxyéthyléné à 33 moles d'oxyde d'éthylène, vendu sous la dénomination "SINNOWAX AO" par la Société HENKEL | 7,0 g |
| - Mélange de monostéarate et de distéarate de glycéryle non autoémulsionnable | 2,0 g |
| - Alcool cétylique | 1,5 g |
| - Huile de silicone | 1,5 g |
| - Huile de vaseline | 15,0 g |
| - Pigment mélanique préparé selon l'exemple 2 | 1,0 g |
| - Glycérine | 20,0 g |
| - Parfum, conservateur qs | |
| - Eau | qsp 100,0 g |

### EXEMPLE 2

On prépare une émulsion solaire huile-dans-eau ayant la composition suivante :

| | |
|---|---|
| - Mélange d'alcool cétylstéarylique et d'alcool cétylstéarylique oxyéthyléné à 33 moles d'oxyde d'éthylène, vendu sous la dénomination "SINNOWAX AO" par la Société HENKEL | 7,0 g |
| - Mélange de monostéarate et de distéarate de glycéryle non autoémulsionnable | 2,0 g |
| - Alcool cétylique | 1,5 g |
| - Huile de silicone | 1,5 g |
| - Huile de vaseline | 15,0 g |
| - Paraméthoxycinnamate de 2-éthyl-hexyle, vendu sous la dénomination "PARSOL MCX" par la Société GIVAUDAN | 5,0 g |
| - Pigment mélanique préparé selon l'exemple 1 | 2,0 g |
| - Glycérine | 20,0 g |
| - Oxyde de fer jaune qs | |
| - Oxyde de fer rouge qs | |
| - Parfum, conservateur qs | |
| - Eau | qsp 100,0 g |

### EXEMPLE 3

On prépare une émulsion solaire huile-dans-eau ayant la composition suivante :

| | |
|---|---|
| - Mélange d'alcool cétylstéarylique et d'alcool cétylstéarylique oxyéthyléné à 33 moles d'oxyde d'éthylène, vendu sous la dénomination "SINNOWAX AO" par la Société HENKEL | 7,0 g |
| - Mélange de monostéarate et de distéarate de glycéryle non autoémulsionnable | 2,0 g |
| - Alcool cétylique | 1,5 g |
| - Huile de silicone | 1,5 g |
| - Huile de vaseline | 15,0 g |
| - Paraméthoxycinnamate de 2-éthyl-hexyle, vendu sous la dénomination "PARSOL MCX" par la Société GIVAUDAN | 5,0 g |
| - Pigment mélanique préparé selon l'exemple 3 | 0,5 g |
| - Glycérine | 20,0 g |
| - Parfum, conservateur qs | |
| - E'au | qsp 100,0 g |

### EXEMPLE 4

On prépare un gel de coloration capillaire ayant la composition suivante :

| | |
|---|---|
| - Pigment mélanique préparé selon l'exemple 1 | 2,0 g |
| - Acide polyacrylique réticulé (PM 4 000 000) vendu sous la dénomination "CARBOPOL 940" par la Société GOODRICH | 0,7 g |
| - Alcool éthylique | 17,0 g |
| - Triéthanolamine qs pH=7,5 | |
| - Eau | qsp 100,0 g |

On applique la composition sur des cheveux à 100% blancs. Après répartition uniforme du produit, les cheveux sont séchés à 60°C pendant environ 15 minutes. Les cheveux sont colorés en gris clair.

### EXEMPLE 5

On prépare une émulsion solaire huile-dans-eau ayant la composition suivante :

| | |
|---|---|
| - Pigment mélanique préparé selon l'exemple 4 | 0,15 g |
| - Oxyde de titane enrobé d'alumine et de stéarate d'aluminium, vendu sous la dénomination "MICRO TiO₂ MT 100T" par la Société TAYCA | 5,0 g |
| - SINNOWAX AO | 7,0 g |
| - Mélange de mono- et distéarate de glycérol non autoémulsionnable | 2,0 g |
| - Alcool cétylique | 1,5 g |
| - Huile de silicone | 1,5 g |
| - Huile de vaseline | 15,0 g |
| - Glycérine | 20,0 g |
| - Triéthanolamine qs pH=7 | |
| - Parfum, conservateurs qs | |
| - Eau purifiée | qsp 100,0 g |

### EXEMPLE 6

On prépare une émulsion ayant la composition suivante :

| | |
|---|---|
| - Pigment mélanique préparé selon l'exemple 3 | 3,0 g |
| - Oxyde de fer jaune | 1,77 g |
| - Oxyde de fer rouge | 0,73 g |
| - Oxyde de titane | 3,5 g |
| - Silicate d'aluminium et de magnésium | 1,0 g |
| - Mélange de mono- et distéarate de glycérol, d'acide stéarique et de glycérine, vendu sous la dénomination "GELEOL Copeaux" par la Société GATTEFOSSE | 2,2 g |
| - Triglycérides d'acides caprique et caprylique | 15,0 g |
| - Acide stéarique | 2,2 g |
| - Triéthanolamine | 1,0 g |
| - Lauroylsarcosinate de sodium | 0,6 g |
| - Diméthylpolysiloxane cyclique | 10,0 g |
| - Sel d'aluminium du produit de réaction de l'anhydride octénylsuccinique et d'amidon | 5,0 g |
| - Carboxyméthylcellulose | 0,16 g |
| - 2-hydroxy 4-méthoxybenzophénone | 0,5 g |
| - Diméthyl p-aminobenzoate de 2-éthylhexyle | 0,5 g |
| - Propylèneglycol | 2,0 g |
| - Glycérine | 3,0 g |
| - Antioxydants, conservateurs qs | |
| - Eau | qsp 100,0 g |

## Revendications

1. Produit sous forme de poudre constitué de particules, caractérisé par le fait que les particules sont des particules à structure lamellaire, organiques ou minérales, ayant une dimension inférieure à 50 microns et comportant au moins un pigment mélanique synthétique, formé in situ par oxydation d'un composé indolique.

2. Produit selon la revendication 1, caractérisé par le fait que le composé indolique répond à la formule (I) : 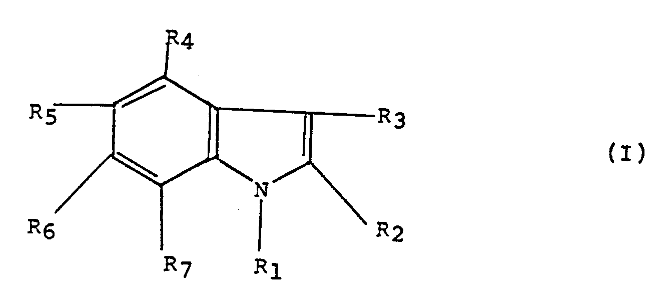 dans laquelle :
R₁ et R₃ désignent, indépendamment l'un de l'autre, un atome d'hydrogène ou un groupe alkyle en C₁-C₄;
R₂ représente un atome d'hydrogène, un groupement alkyle en C₁-C₄, un groupement carboxyle ou un groupement alcoxy(C₁-C₄)carbonyle;
R₄ et R₇ désignent, indépendamment l'un de l'autre, un atome d'hydrogène, un groupement hydroxy, un groupement alkyle en C₁-C₄, amino, alcoxy(C₁-C₄), acyl (C₂-C₄)oxy, acyl(C₂-C₄)amino;
R₅ désigne un atome d'hydrogène, un groupement hydroxy, alcoxy(C₁-C₄), alkyle(C₁-C₄), halogène, amino, acyl(C₂-C₁₄)oxy, acyl(C₂-C₄)amino, triméthylsilyloxy;
R₆ désigne un atome d'hydrogène, un groupement hydroxy, alcoxy(C₁-C₄), amino, acyl(C₂-C₄)oxy, acyl (C₂-C₄)amino, triméthylsilyloxy, hydroxyalkyl(C₂-C₄) amino;
R₅ et R₆ pouvant former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy;
au moins l'un des groupements R₄ à R₇ représente un groupement OZ ou NHR, un seul au plus des groupements R₄ à R₇ désignant NHR;
et au plus deux des groupements R₄ à R₇ désignent OZ, ces groupements étant en position 5 et 6 lorsque Z désigne hydrogène, et au moins l'un des groupements R₄ à R₇ représente un atome d'hydrogène et dans ce cas, si un seul de ces groupements désigne hydrogène, un seul groupement parmi R₄ à R₇ désigne alors NHR ou OZ, les autres groupements désignant alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants de ces composés.

3. Produit selon les revendications 1 ou 2, caractérisé par le fait que le composé indolique est choisi parmi les composés suivants : le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxy indole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxy carbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, 5-aminoindole, 6-aminoindole, 7-amino indole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole.

4. Produit selon l'une quelconque des revendications 1 à 3, caractérisé par le fait que le composé indolique est le 5,6-dihydroxyindole ou le 6-hydroxyindole ou un mélange des deux.

5. Produit selon l'une quelconque des revendications 1 à 4, caractérisé par le fait que les particules à structure lamellaire sont choisies parmi la L-lauroyllysine, les microparticules de céramique éventuellement recouvertes de poudre de zirconium, le dioxyde de titane lamellaire, le talc lamellaire, le nitrure de bore, le mica, l'oxychlorure de bismuth, l'oxyde de fer transparent rouge.

6. Produit selon l'une quelconque des revendications 1 à 5, caractérisé par le fait que les particules à structure lamellaires sont constituées de feuillets pour lesquels le rapport entre la plus grande dimension et l'épaisseur est compris entre 2 et 100.

7. Produit selon l'une quelconque des revendications 1 à 6, caractérisé par le fait que les particules à structure lamellaire ont une dimension supérieure à 0,5 micron et inférieure à 50 microns et de préférence inférieure à 25 microns.

8. Procédé de préparation d'un produit tel que défini dans l'une quelconque des revendications 1 à 7, caractérisé par le fait que l'on mélange dans un milieu aqueux un composé indolique tel que défini dans la revendication 2 ou 3 et des particules à structure lamellaire organiques ou minérales, ayant une dimension inférieure à 50 microns et qu'on procède ensuite à la formation du pigment mélanique par oxydation du composé indolique de formule (I).

9. Procédé selon la revendication 8, caractérisé par le fait que l'oxydation s'effectue lentement à l'air à pH alcalin.

10. Procédé selon la revendication 8, caractérisé par le fait que l'oxydation s'effectue par l'oxygène en présence d'un catalyseur métallique.

11. Procédé selon la revendication 8, caractérisé par le fait que l'oxydation s'effectue par l'addition d'agents oxydants constitués par le peroxyde d'hydrogène, l'acide periodique et ses sels, les permanganates, les bichromates, l'hypochlorite de sodium, le ferricyanure de potassium, le persulfate d'ammonium, l'oxyde d'argent, l'oxyde de plomb, le chlorure ferrique, le nitrite de sodium, les sels de terres rares et les oxydants organiques choisis parmi les ortho et parabenzoquinones, les ortho et parabenzoquinones monoimines ou diimines, les 1,2 et 1,4-naphtoquinones, les 1,2 et 1,4-naphtoquinones monoou diimines.

12. Procédé selon la revendication 8, caractérisé par le fait que l'oxydation s'effectue par le peroxyde d'hydrogène en milieu ammoniacal.

13. Procédé selon l'une quelconque des revendications 7 à 12, caractérisé par le fait que le milieu réactionnel est un milieu essentiellement non solvant des particules lamellaires et solvant du composé indolique de formule (I), et qu'il est constitué par de l'eau ou un mélange d'eau et de solvant(s).

14. Procédé selon l'une quelconque des revendications 8 à 13, caractérisé par le fait que le solvant est choisi parmi l'alcool éthylique, l'alcool propylique ou isopropylique, l'alcool tertiobutylique, les alkylèneglycols, les alkyléthers d'alkylèneglycols et le lactate de méthyle.

15. Procédé selon l'une quelconque des revendications 8 à 14, caractérisé par le fait que le composé indolique est utilisé dans des proportions en poids comprises entre 0,1 et 10% et de préférence entre 0,5 et 5% en poids, par rapport au poids du milieu réactionnel et que les particules à structure lamellaire, organiques ou minérales, sont utilisées dans des proportions comprises entre 0,05 et 35% en poids.

16. Composition cosmétique, caractérisée par le fait qu'elle contient dans un milieu cosmétiquement acceptable, au moins un produit tel que défini dans l'une quelconque des revendications 1 à 7, ou préparé selon l'une quelconque des revendications 8 à 15.

17. Composition selon la revendication 16, caractérisée par le fait qu'elle se présente sous forme de lotion, de lotion épaissie, de gel, de crème, de lait, de poudre, de stick, et qu'elle est éventuellement conditionnée en aérosol sous forme de spray ou de mousse.

18. Composition selon l'une quelconque des revendications 16 et 17, caractérisée par le fait qu'elle est destinée à être utilisée pour le maquillage de la peau, des cils et des sourcils, et qu'elle se présente sous forme solide ou pâteuse, anhydre ou aqueuse.

19. Composition selon l'une quelconque des revendications 16 et 17, destinée à la protection de l'épiderme humain contre les rayonnements solaires UV, caractérisée par le fait qu'elle se présente sous forme de suspension ou de dispersion dans des solvants ou des corps gras, ou sous forme d'émulsions, de pommades, de gels, de bâtonnets solides ou de mousses aérosols.

20. Composition selon l'une quelconque des revendications 16 à 19, caractérisée par le fait qu'elle contient des corps gras, des solvants organiques, des silicones, des épaississants, des adoucissants, des tensio-actifs, des filtres solaires, des agents anti-mousses, des agents hydratants, des parfums, des conservateurs, des agents anti-oxydants, des charges, des séquestrants, des agents de traitement, des propulseurs, des agents alcalinisants ou acidifiants ou d'autres pigments.

21. Composition selon la revendication 20, caractérisée par le fait qu'elle contient des nanopigments d'oxydes métalliques choisis parmi les oxydes de titane, de zinc, de cérium ou de zirconium, ces nanopigments ayant un diamètre moyen inférieur à 100 nm, et sont enrobés ou non enrobés.

22. Composition selon l'une quelconque des revendications 16 à 21, caractérisée par le fait que la poudre sous forme de particules lamellaires comportant le pigment mélanique est présente dans des concentrations comprises entre 0,1 et 35% en poids et de préférence entre 0,5 et 20% en poids par rapport au poids total de la composition.

23. Utilisation du produit tel que défini dans l'une quelconque des revendications 1 à 7 ou préparé selon le procédé défini dans l'une quelconque des revendications 8 à 15, dans la protection de l'épiderme humain contre le rayonnement UV.

24. Utilisation du produit tel que défini dans l'une quelconque des revendications 1 à 7 ou préparé selon le procédé défini dans l'une quelconque des revendications 8 à 15, dans des produits de maquillage.

25. Utilisation du produit tel que défini dans l'une quelconque des revendications 1 à 7 ou préparé selon le procédé défini dans l'une quelconque des revendications 8 à 15, dans des compositions de teinture des cheveux humains.

## Claims

1. Product in the form of powder consisting of particles, characterized in that the particles are organic or inorganic particles having a lamellar structure, having a size of less than 50 microns and containing at least one synthetic melanin pigment formed in situ by oxidation of an indole compound.

2. Product according to Claim 1, characterized in that the indole compound corresponds to the formula (I): 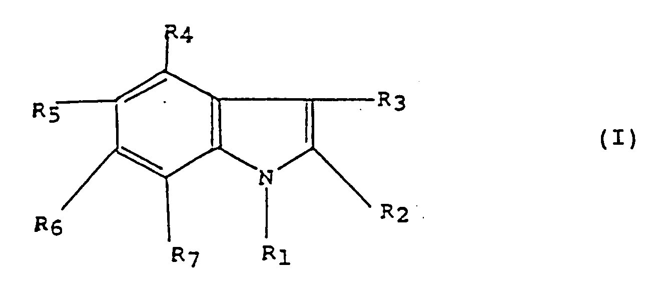 in which:
R₁ and R₃ denote, independently of one another, a hydrogen atom or a C₁-C₄ alkyl group;
R₂ represents a hydrogen atom, a C₁-C₄ alkyl group, a carboxyl group or a (C₁-C₄)alkoxycarbonyl group;
R₄ and R₇ denote, independently of one another, a hydrogen atom, a hydroxyl group or a C₁-C₄ alkyl, amino, (C₁-C₄) alkoxy, (C₂-C₄) acyloxy or (C₂-C₄) acylamino group;
R₅ denotes a hydrogen atom or a hydroxyl, (C₁-C₄) alkoxy, (C₁-C₄) alkyl, halogen, amino, (C₂-C₁₄) acyloxy, (C₂-C₄) acylamino or trimethylsilyloxy group;
R₆ denotes a hydrogen atom or a hydroxyl, (C₁-C₄) alkoxy, amino,(C₂-C₄) acyloxy, (C₂-C₄), acylamino, trimethylsilyloxy or hydroxy(C₂-C₄)alkylamino group;
it being possible for R₅ and R₆ to form, conjointly with the carbon atoms to which they are attached, a methylenedioxy ring, which may be substituted by a C₁-C₄ alkyl or C₁-C₄ alkoxy group or a carbonyldioxy ring;
at least one of the groups R₄ to R₇ represents an OZ or NHR group, and at most one of the groups R₄ to R₇ denotes NHR;
and at most two of the groups R₄ to R₇ denote OZ, these groups being in the 5- and 6- positions when Z denotes hydrogen, and at least one of the groups R₄ to R₇ represents a hydrogen atom and in this case, if only one of these groups denotes hydrogen, only one group from R₄ to R₇ then denotes NHR or OZ, the other groups denoting C₁-C₄ alkyls;
R in NHR denoting a hydrogen atom or a C₂-C₄ acyl or C₂-C₄ hydroxyalkyl group and Z in OZ denoting a hydrogen atom or a C₂-C₁₄ acyl, C₁-C₄ alkyl or trimethylsilyl group;
and the corresponding salts of these compounds.

3. Product according to Claims 1 or 2, characterized in that the indole compound is chosen from the following compounds: 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole. 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindole, 7-hydroxy-3-methylindole, 5-hydroxy-6-methoxy-2,3-dimethylindole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxycarbonyl-5-methoxyindole, 6-N-β-hydroxyethylaminoindole, 4-aminoindole, 5-aminoindole, 6-aminoindole, 7-aminoindole, N-methyl-6-β-hydroxyethylaminoindole, 6-amino-2,3-dimethylindole, 6-amino-2,3,4,5-tetramethylindole, 6-amino-2,3,4-trimethylindole, 6-amino-2,3,5-trimethylindole, 6-amino-2,3,6-trimethylindole, 5,6-diacetoxyindole, 5-methoxy-6-acetoxyindole and 5,6-dimethoxyindole.

4. Product according to any one of Claims 1 to 3, characterized in that the indole compound is 5,6-dihydroxyindole or 6-hydroxyindole or a mixture of the two.

5. Product according to any one of Claims 1 to 4, characterized in that the particles having a lamellar structure are chosen from L-lauroyllysine, ceramic microparticles optionally covered with zirconium powder, lamellar titanium dioxide, lamellar talc, boron nitride, mica, bismuth oxychloride and red transparent iron oxide.

6. Product according to any one of Claims 1 to 5, characterized in that the particles having a lamellar structure consist of thin plates for which the ratio between the largest dimension and the thickness is between 2 and 100.

7. Product according to any one of Claims 1 to 6, characterized in that the particle having a lamellar structure have a size of more than 0.5 micron and less 50 microns and preferably less than 25 microns.

8. Process for the preparation of a product as defined in any one of Claims 1 to 7, characterized in that an indole compound as defined in Claim 2 or 3 and organic or inorganic particles having a lamellar structure and having a size of less than 50 microns are mixed in an aqueous medium and in that the formation of the melanin pigment is then carried out by oxidation of the indole compound of formula (I).

9. Process according to Claim 8, characterized in that the oxidation takes place slowly in air at alkaline pH.

10. Process according to Claim 8, characterized in that the oxidation takes place by means of oxygen in the presence of a metal catalyst.

11. Process according to Claim 8, characterized in that the oxidation takes place by the addition of oxidizing agents consisting of hydrogen peroxide, periodic acid and its salts, permanganates, bichromates, sodium hypochlorite, potassium ferricyanide, ammonium persulphate, silver oxide, lead oxide, ferric chloride, sodium nitrite, rare earth salts and organic oxidizing agents chosen from ortho- and para-benzoquinones, ortho- and para-benzoquinone monoimines or diimines, 1,2- and 1,4-naphthoquinones and 1,2- and 1,4-naphthoquinone monoimines or diimines.

12. Process according to Claim 8, characterized in that the oxidation takes place by means of hydrogen peroxide in an ammoniacal medium.

13. Process according to any one of Claims 7 to 12, characterized in that the reaction medium is a medium which is essentially a non-solvent for the lamellar particles and a solvent for the indole compound of formula (I) and in that said medium consists of water or a mixture of water and solvent(s).

14. Process according to any one of Claims 8 to 13, characterized in that the solvent is chosen from ethyl alcohol, propyl or isopropyl alcohol, tert-butyl alcohol, alkylene glycols, alkylene glycol alkyl ethers and methyl lactate.

15. Process according to any one of Claims 8 to 14, characterized in that the indole compound is used in proportions by weight of between 0.1 and 10% and preferably between 0.5 and 5% by weight relative to the weight of the reaction mixture, and in that the organic or inorganic particles having a lamellar structure are used in proportions of between 0.05 and 35% by weight.

16. Cosmetic composition, characterized in that it contains, in a cosmetically acceptable medium, at least one product as defined in any one of Claims 1 to 7 or prepared in accordance with any one of Claims 8 to 15.

17. Composition as claimed in Claim 16, characterized in that it is in the form of a lotion, thickened lotion, gel, cream, milk, powder or stick and in that it is optionally packaged in an aerosol in the form of a spray or foam.

18. Composition according to either of Claims 16 and 17, characterized in that it is intended to be used for making up the skin, the eyelashes and the eyebrows and in that it is in anhydrous or aqueous solid or pasty form.

19. Composition according to either one of Claims 16 and 17, intended to protect the human epidermis against solar UV radiation, characterized in that it is in the form of a suspension or dispersion in solvents or fats, or in the form of emulsions, ointments, gels, solid sticks or aerosol foams.

20. Composition according to any one of Claims 16 to 19, characterized in that it contains fats, organic solvents, silicones, thickeners, emollients, surfactants, sun filters, anti-foams, hydrating agents, perfumes, preservatives, antioxidants, fillers, sequestering agents, treatment agents, propellants, alkalinizing or acidifying agents or other pigments.

21. Composition according to Claim 20, characterized in that it contains metal oxide nanopigments chosen from titanium oxide, zinc oxide, cerium oxide or zirconium oxide, these nanopigments having an average diameter of less than 100 nm and are coated or non-coated.

22. Composition according to one of Claims 16 to 21, characterized in that the powder in the form of lamellar particles containing the melanin pigment is present in concentrations of between 0.1 and 35% by weight and preferably between 0.5 and 20% by weight, relative to the total weight of the composition.

23. Use of the product as defined in any one of Claims 1 to 7 or prepared in accordance with the process defined in any one of Claims 8 to 15 in the protection of the human epidermis against UV radiation.

24. Use of the product as defined in any one of Claims 1 to 7 or prepared in accordance with the process defined in any one of Claims 8 to 15, in make-up products.

25. Use of the product as defined in any one of Claims 1 to 7 or prepared in accordance with the process defined in any one of Claims 8 to 15 in compositions for dyeing human hair.

## Patentansprüche

1. Produkt in Form eines Pulvers aus Partikeln,
dadurch **gekennzeichnet**, daß
die Partikel organische oder mineralische Partikel mit plättchenförmiger Struktur sind, die in Abmessungen unterhalb 50 »m vorliegen und mindestens ein synthetisches Melaninpigment enthalten, das in situ durch Oxidation einer Indolverbindung gebildet ist.

2. Produkt gemäß Anspruch 1,
dadurch **gekennzeichnet**, daß
die Indolverbindung durch die Formel (I): 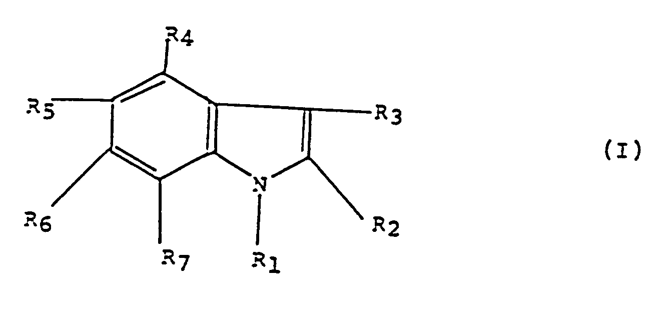 worin gilt:
R₁ und R₃ bezeichnen, unabhängig voneinander, ein Wasserstoffatom oder eine C₁₋₄-Alkylgruppe;
R₂ stellt ein Wasserstoffatom, eine C₁₋₄-Alkylgruppe, eine Carboxyl- oder C₁₋₄-Alkoxycarbonylgruppe dar;
R₄ und R₇ bezeichnen, unabhängig voneinander, ein Wasserstoffatom, eine Hydroxy-, C₁₋₄-Alkyl-, Amino-, C₁₋₄-Alkoxy-, C₂₋₄-Acyloxy- oder C₂₋₄-Acylaminogruppe;
R₅ bedeutet Wasserstoff, eine Hydroxy-, C₁₋₄-Alkoxy-, C₁₋₄-Alkyl-, Halogen-, Amino-, C₂₋₄-Acyloxy-, C₂₋₄-Acylamino- oder Trimethylsilyloxygruppe;
R₆ bedeutet Wasserstoff, eine Hydroxy-, C₁₋₄-Alkoxy-, Amino-, C₂₋₄-Acyloxy-, C₂₋₄-Acylamino-, Trimethylsilyloxy- oder C₂₋₄-Hydroxyalkylaminogruppe;
R₅ und R₆ können zusammen mit den Kohlenstoffatomen, an die sie gebunden sind, einen Methylendioxiring, der gegebenenfalls mit einer C₁₋₄-Alkyl- oder C₁₋₄-Alkoxygruppe substituiert ist, oder einen Carbonyldioxiring bilden;
mindestens eine der Gruppen R₄ bis R₇ stellt eine OZ- oder NHR-Gruppe dar, wobei höchstens eine einzige der Gruppen R₄ bis R₇ NHR bedeutet; und höchstens zwei der Gruppen R₄ bis R₇ bedeuten OZ, wobei, wenn Z Wasserstoff bedeutet, diese Gruppen in Position 5 und 6 vorliegen, und mindestens eine der Gruppen R₄ bis R₇ stellt Wasserstoff dar, und, wenn eine einzige dieser Gruppen Wasserstoff bedeutet, stellt eine einzige Gruppe aus R₄ bis R₇ dann NHR oder OZ dar, wobei die anderen Gruppen eine C₁₋₄-Alkylgruppe bedeuten; wobei R in der NHR-Gruppe ein Wasserstoffatom, eine C₂₋₄-Acyl-, C₂₋₄-Hydroxyakylgruppe und Z in der OZ-Gruppe ein Wasserstoffatom, eine C₂₋₁₄-Acyl-, C₁₋₄-Alkyl- oder Trimethylsilylgruppe darstellen;
sowie durch die entsprechenden Salze dieser Verbindungen dargestellt ist.

3. Produkt gemäß Anspruch 1 oder 2,
dadurch **gekennzeichnet**, daß
die Indolverbindung aus den folgenden Verbindungen ausgewählt ist:
4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5-6-Dihydroxyindol, N-Methyl-5,6-dihydroxyindol, 2-Methyl-5,6-dihydroxyindol, 3-Methyl-5,6-dihydroxyindol, 2,3-Dimethyl-5,6-dihydroxyindol, 2-Carboxy-5,6-dihydroxyindol, 4-Hydroxy-5-methylindol, 2-Carboxy-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxy-5-methylindol, 6-Hydroxy-2-ethoxycarbonyl-5-methoxyindol, 6-N-β-Hydroxyethylaminoindol, 4-Aminoindol, 5-Aminoindol, 6-Aminoindol, 7-Aminoindol, N-Methyl-6-β-hydroxyethylaminoindol, 6-Amino-2,3-dimethylindol, 6-Amino-2,3,4,5-tetramethylindol, 6-Amino-2,3,4-trimethylindol, 6-Amino-2,3,5-trimethylindol, 6-Amino-2,3,6-trimethylindol, 5,6-Diacetoxyindol, 5-Methoxy-6-acetoxyindol, 5,6-Dimethoxyindol.

4. Produkt gemäß jedem der Ansprüche 1 bis 3,
dadurch **gekennzeichnet**, daß
die Indolverbindung 5,6-Dihydroxyindol oder 6-Hydroxyindol oder eine Mischung der beiden ist.

5. Produkt gemäß jedem der Ansprüche 1 bis 4,
dadurch **gekennzeichnet**, daß
die Partikel mit plättchenförmiger Struktur aus L-Lauroyllysin, gegebenenfalls mit Zirkonpulver bedeckten Keramikmikropartikeln, plättchenförmigem Titandioxid, plättchenförmigem Talk, Bornitrid, Glimmer, Wismutoxichlorid, durchsichtigem roten Eisenoxid ausgewählt sind.

6. Produkt gemäß jedem der Ansprüche 1 bis 5,
dadurch **gekennzeichnet**, daß
die Partikel mit plättchenförmiger Struktur aus Blättern zusammengesetzt sind, für die das Verhältnis zwischen der größten Abmessung und der Dicke 2 bis 100 beträgt.

7. Produkt gemäß jedem der Ansprüche 1 bis 6,
dadurch **gekennzeichnet**, daß
die Partikel mit plättchenförmiger Struktur Abmessungen oberhalb 0,5 »m und unterhalb 50 »m, vorzugsweise unterhalb 25 »m aufweisen.

8. Verfahren zur Herstellung eines in jedem der Ansprüche 1 bis 7 definierten Produkts,
dadurch **gekennzeichnet**, daß
man in einem wässrigen Milieu eine in Anspruch 2 oder 3 definierte Indolverbindung und organische oder mineralische Partikel mit plättchenförmiger Struktur, die eine Größe unterhalb 50 »m aufweisen, vermischt und sodann das Melaninpigment durch Oxidation der Indolverbindung der Formel (I) bildet.

9. Verfahren gemäß Anspruch 8,
dadurch **gekennzeichnet**, daß
die Oxidation langsam an Luft bei alkalischem pH erfolgt.

10. Verfahren gemäß Anspruch 8,
dadurch **gekennzeichnet**, daß
die Oxidation durch Sauerstoff in Gegenwart eines metallhaltigen Katalysators erfolgt.

11. Verfahren gemäß Anspruch 8,
dadurch **gekennzeichnet**, daß
die Oxidation durch Zugabe von Oxidationsmitteln aus Wasserstoffperoxid, Perjodsäure und ihren Salzen, Permanganaten, Bichromaten, Natriumhypochlorit, Kaliumferricyanid, Ammoniumpersulfat, Silberoxid, Bleioxid, Eisen(III)chlorid, Natriumnitrit, aus Salzen von seltenen Erden, aus organischen Oxidiermitteln erfolgt, ausgewählt aus o- und p-Benzochinonen, o- und p-Benzochinonmonoiminen oder - diiminen, 1,2- und 1,4-Naphthochinonen, 1,2- und 1,4-Naphthochinonmono- oder -diiminen.

12. Verfahren gemäß Anspruch 8,
dadurch **gekennzeichnet**, daß
die Oxidation durch Wasserstoffperoxid in ammoniakalischem Milieu erfolgt.

13. Verfahren gemäß jedem der Ansprüche 7 bis 12,
dadurch **gekennzeichnet**, daß
das Reaktionsmilieu ein Milieu ist, das im wesentlichen ein Nicht-Lösungsmittel für die plättchenförmigen Partikel und ein Lösungsmittel für die Indolverbindung der Formel (I) ist, und daß es aus Wasser oder einer Mischung aus Wasser und Lösungsmittel(n) zusammengesetzt ist.

14. Verfahren gemäß jedem der Ansprüche 8 bis 13,
dadurch **gekennzeichnet**, daß
das Lösungsmittel aus Ethyl-, Propyl- oder Isopropyl-, t-Butylalkohol, Alkylenglycolen, Alkylethern von Alkylenglycolen und Methyllactat ausgewählt ist.

15. Verfahren gemäß jedem der Ansprüche 8 bis 14,
dadurch **gekennzeichnet**, daß
die Indolverbindung in Mengenverhältnissen von 0,1 bis 10 Gew.%, vorzugsweise 0,5 bis 5 Gew.%, bezogen auf das Gesamtgewicht des Reaktionsmilieu, und die organischen oder mineralischen Partikel mit plättchenförmiger Struktur in Mengenverhältnissen von 0,05 bis 35 Gew.% verwendet werden.

16. Kosmetische Zusammensetzung,
dadurch **gekennzeichnet**, daß
sie in einem kosmetisch verträglichen Milieu mindestens ein in jedem der Ansprüche 1 bis 7 definiertes oder gemäß jedem der Ansprüche 8 bis 15 hergestelltes Produkt enthält.

17. Zusammensetzung gemäß Anspruch 16,
dadurch **gekennzeichnet**, daß
sie in Form einer Lotion, verdickten Lotion, eines Gels, einer Creme, einer Milch, eines Pulvers oder eines Stifts vorliegt und daß sie ggf. als ein Aerosol in Form eines Spray oder Schaums zubereitet ist.

18. Zusammensetzung gemäß jedem der Ansprüche 16 und 17,
dadurch **gekennzeichnet**, daß
sie zur Verwendung zum Schminken der Haut, Wimpern und Augenbrauen bestimmt ist und in fester oder pasteuser, wasserfreier oder wässriger Form vorliegt.

19. Zusammensetzung gemäß jedem der Ansprüche 16 und 17 zum Schutz der menschlichen Epidermis vor UV-Sonnenstrahlen,
dadurch **gekennzeichnet**, daß
sie in Form einer Suspension oder Dispersion in Lösungsmitteln oder Fettkörpern oder in Form von Emulsionen, Pommaden, Gelen, Feststoffstäbchen oder Aerosol-Schäumen vorliegt.

20. Zusammensetzung gemäß jedem der Ansprüche 16 bis 19,
dadurch **gekennzeichnet**, daß
sie Fettkörper, organische Lösungsmittel, Silikone, Verdickungsmittel, Weichmacher, oberflächenaktive Mittel, Sonnenfilterstoffe, Antischaummittel, Hydratisiermittel, Parfüm-Produkte, Konservierungsstoffe, Antioxidantien, Chargiermittel, Sequestriermittel, Behandlungsmittel, Treibmittel, alkalisch oder sauer machende Mittel oder weitere Pigmente enthält.

21. Zusammensetzung gemäß Anspruch 20,
dadurch **gekennzeichnet**, daß
sie Nanopigmente aus Metalloxiden enthält, die aus Oxiden von Titan, Zink, Cer oder Zirkon ausgewählt sind, wobei diese Nanopigmente einen Durchmesser unterhalb 100 nm aufweisen und umhüllt oder nicht umhüllt sind.

22. Zusammensetzung gemäß jedem der Ansprüche 16 bis 21,
dadurch **gekennzeichnet**, daß
das Pulver in Form von das Melaninpigment enthaltenden plättchenförmigen Partikeln in Konzentrationen von 0,1 bis 35 Gew.%, vorzugsweise von 0,5 bis 20 Gew.%, vorhanden ist, bezogen auf das Gesamtgewicht der Zusammensetzung.

23. Verwendung des in jedem der Ansprüche 1 bis 7
definierten oder gemäß dem in jedem der Ansprüche 8 bis 15 definierten Verfahren hergestellten Produkts zum Schutz der menschlichen Epidermis vor UV-Strahlung.

24. Verwendung des in jedem der Ansprüche 1 bis 7
definierten oder gemäß dem in jedem der Ansprüche 8 bis 15 definierten Verfahren hergestellten Produkts in Schmink-Produkten.

25. Verwendung des in jedem der Ansprüche 1 bis 7
definierten oder gemäß dem in jedem der Ansprüche 8 bis 15 deifnierten Verfahren hergestellten Produkts in Zusammensetzungen zur Färbung menschlicher Haare.
